(19) Europäisches Patentamt

European Patent Office

Office européen des brevets

(11) **EP 0 792 646 B1**

(12) **FASCICULE DE BREVET EUROPEEN**

(45) Date de publication et mention
de la délivrance du brevet:
**22.05.2002 Bulletin 2002/21**

(51) Int Cl.7: **A61K 35/78**, A61K 7/48,
A61K 7/06

(21) Numéro de dépôt: **97400387.3**

(22) Date de dépôt: **21.02.1997**

(54) **Compositions cosmétiques ou dermopharmaceutiques contenant un extrait de Solanum lycocarpum**

Kosmetische und dermopharmazeutische Zusammensetzungen, enthaltend einen Solanum lycocarpum Extrakt

Cosmetic or dermopharmaceutical composition containing an extract from Solanum lycocarpum

(84) Etats contractants désignés:
**AT BE CH DE DK ES FI GB GR IE IT LI LU NL PT SE**

(30) Priorité: **22.02.1996 FR 9602318**

(43) Date de publication de la demande:
**03.09.1997 Bulletin 1997/36**

(73) Titulaire: **CLARINS**
**F-92203 Neuilly-sur-Seine Cédex (FR)**

(72) Inventeur: **Greff, Daniel**
**78490 Mere (FR)**

(74) Mandataire: **Rousset, Jean-Claude et al**
**Cabinet Netter**
**36, avenue Hoche**
**75008 Paris (FR)**

(56) Documents cités:
• **BOLETIM DA SOCIEDADE BROTERIANA, vol. 58, no. 2, 1985, pages 43-56, XP000609377 LAURO XAVIER FILHO ET AL.: "CONTRIBUIC O AO ESTUD DE PLANTAS MEDICINAIS DO CERRADO, BRASIL CENTRAL"**
• **AN. ACAD. BRASIL. CI NC., vol. 42, no. SUPLEMENTO, 1970, pages 375-376, XP000609385 M. MOTIDOME ET AL.: "A QU MICA DE SOLAN CEAS BRASILEIRAS. I- A PRESENCA DE SOLAMARGINA E DE SOLASONINA NO JU E NA LOBEIRA."**

## Description

La plante brésilienne appelée Fruta de lobo, du nom scientifique *Solanum lycocarpum* est un petit arbuste épineux qui donne un fruit non comestible. Alors que la population indigène de la région de récolte (Minas Gérais) utilise les feuilles pour préparer des infusions, le fruit frais ou séché n'est pas utilisé actuellement.

Dans le Boletim da Sociedade Broteriana (Vol. 58, No. 2, 1985 : 43-56) Solanum lycocarpum est décrite comme n'étant pas de grand intérêt médical. Elle est sédative, utilisée dans les maladies des passages urinaines et dans les états nerveux.

L'objet du présent brevet est la découverte que certains extraits obtenus à partir de cette plante, surtout du fruit, possèdent des activités biochimiques utiles pour l'application cosmétique et dermopharmaceutique à savoir, le pouvoir de stimuler la respiration mitochondriale, de stimuler la prolifération cellulaire en culture, d'inhiber l'action délétère des radicaux libres et d'inhiber la synthèse de mélanine.

L'avantage des extraits de ce fruit réside dans un ratio favorable d'activité biologique sur matière sèche (et couleur associée), ce qui est souvent problématique en phytothérapie. Les extraits sont stables, sans forte odeur et facilement utilisés dans tous type de produit cosmétique.

A titre d'exemple nous décrivons une extraction de *Solanum lycocarpum* qui permet d'extraire les principes d'activité cosmétique sans trop colorer l'extrait final.

200 g de fruits secs sont macérés pendant 2 heures dans 500 ml d'éthanol pur. On élimine cette fraction éthanolique, puis on fait macérer le résidu dans 1500 ml d'un mélange de butylène glycol (40%), de glycérine (40%) et d'eau (20%). Après différents stades de filtration on récupère un filtrat jaune limpide que l'on décolore sur cartouche charbon actif.

On obtient un produit limpide, clair, stable, riche en substances d'intérêt cosmétique : acides organiques, vitamine C, dérivés galliques, sucres.

Les extraits d'autres espèces de Solanacées sont décrits dans la littérature et dans certains brevets : *Solanum tuberosum* (pomme de terre), *Solanum lycopersicum* (tomate), *Solanum melongena* (aubergine) etc...

Dans ces extraits on ne décrit pas les activités cosmétiques que nous avons trouvées dans Fruta de lobo.

Les solvants d'extraction pour la préparation des extraits objets du présent brevet peuvent être choisis parmi l'eau, le propylène glycol, le butylène glycol, la glycérine, les polyéthylènes glycols, les éthers méthyliques ou éthyliques des diglycols, les polyols cycliques, les diglycols éthoxylés ou propoxylés ou tout mélange de ces solvants.

L'exemple donné de fabrication de l'extrait n'est pas limitatif. Les améliorations, optimisations et modifications du procédé envisageables sont évidentes pour l'homme de l'art. A la place de la macération simple, on peut employer des techniques à contre courant, la décoction, la lixiviation, l'extraction à l'aide d'ultrasons, de micro-ondes associée ou non aux solvants.

Pour certaines applications cosmétiques il peut s'avérer utile de préparer un extrait sec à partir de l'extrait liquide. Ceci peut être réalisé par les techniques classiques de séchage, d'évaporation, d'atomisation ou de lyophilisation.

L'activité biochimique la plus évidente des extraits de Fruta de lobo est le pouvoir stimulateur de la respiration cellulaire et de la prolifération des fibroblastes en culture.

Un exemple illustre cet effet: Stimulation de la respiration cellulaire (OXYGRAPHIE).

Le principe et les détails de la méthode ont été très largement décrits précédemment. A l'aide d'une électrode de type CLARK et d'un oxygraphe GILSON™, on mesure le taux de consommation d'oxygène de base d'une suspension de cellules hépatiques. L'introduction du produit actif dans la suspension stimule la respiration mitochondriale, ce qui se traduit par une augmentation de la consommation d'oxygène. L'enregistrement des concentrations d'oxygène dissout dans le milieu en fonction du temps donne des courbes linéaires inclinées.

L'augmentation de la pente d'inclinaison s'exprime en pourcentage d'après la formule :

$$\frac{n_e - n_t}{n_t} \times 100$$

$n_t$ : nombre de moles d'oxygène consommées par minute et par mg de cellules fraîches pour le lot témoin.

$n_e$ : nombre de moles d'oxygène consommées par minute et par mg de cellules fraîches pour le lot essai.

La dose de 2 % de l'extrait décrit dans cette expérience conduit à une augmentation de la respiration cellulaire de 131% ± 4%.

Stimulation de la prolifération cellulaire

Des fibroblastes 3T3 sont ensemencés sur boîte de culture en présence de DMEM et de 10% de sérum de veau foetal, incubés pendant 24 heures. On rince ensuite et on change le milieu en le différenciant :

DMEM + 10% de sérum: milieu de culture optimal
DMEM + 2% de sérum: milieu de culture suboptimal
DMEM + 2% de sérum + 3% d'un extrait aqueux de Fruta de lobo: milieu test

Les cellules sont trypsinisées après 72 heures et dénombrées sous microscope inverse (NIKON)™.
L'addition de 3% de l'extrait aqueux de Fruta de Lobo dans le milieu suboptimal permet de stimuler la croissance et la prolifération cellulaire au taux de 46 ± 5% par rapport au milieu suboptimal de 2% de sérum seul. Les extraits ainsi obtenus peuvent être employés dans les produits cosmétiques et dermopharmaceutiques de toute sorte. De telles compositions présentent des activités anti-vieillissement, favorisent ou régulent le renouvellement cellulaire cutané, protègent contre la chute des cheveux et possèdent des activités anti-inflammatoires et anti-radicalaires. Certains extraits de Fruta de lobo possèdent une activité dépigmentante de la peau.

Leur utilisation principale est la cosmétique de soins anti-âge et antirides. En outre, les effets toniques de l'extrait contribuent à une restructuration de l'épiderme, améliorent l'aspect de la peau, diminuent la microcirculation capillaire (couperose), donnent élasticité et fermeté à la peau fatiguée.

Les extraits de *Solanum lycocarpum* objets du présent brevet peuvent trouver application également dans les traitements du cuir chevelu (antipelliculaire) ou de la peau acnéique.

Les extraits de *Solanum lycocarpum* objet du présent brevet peuvent être utilisés dans toute forme galénique employée en cosmétique: émulsions H/E et E/H, laits, lotions, gels, pommades, huiles corporelles, lotions capillaires, shampooings, savons, sticks et crayons, sprays, sans que cette liste soit limitative.

Il est possible d'incorporer les extraits de *Solanum lycocarpum* décrit dans des vecteurs cosmétiques comme les liposomes, les chylomicrons, les macro-, micro- et nanoparticules ainsi que les macro-, micro- et nanocapsules, de les absorber sur des polymères organiques poudreux, les talcs, bentonites et autres supports minéraux.

La concentration d'utilisation de ces extraits de *Solanum lycocarpum* peut varier entre 0.1 et 30% (p/p), préférentiellement entre 0.5 et 10% en poids de la composition totale.

Les extraits de *Solanum lycocarpum* peuvent être combinés dans les compositions cosmétiques avec tout autre ingrédient habituellement utilisé en cosmétique : lipides d'extraction et/ou de synthèse, polymères gélifiants et viscosants, tensioactifs et émulsifiants, principes actifs hydro- ou liposolubles, extraits d'autres plantes, extraits tissulaires, extraits marins.

## Revendications

1.  Compositions cosmétiques ou dermopharmaceutiques à activité anti-vieillissement, favorisant ou régulant le renouvellement cellulaire cutané, protégeant contre la chute des cheveux et possédant des activités anti-inflammatoires et anti-radicalaires **caractérisées en ce qu'**elles contiennent un extrait de *Solanum lycocarpum*.

2.  Compositions cosmétiques ou dermopharmaceutiques selon la revendication 1 **caractérisées en ce que** l'extrait de *Solanum lycocarpum* pré-cité est obtenu à partir de la plante entière ou de préférence à partir des fruits frais ou secs de *Solanum lycocarpum*.

3.  Compositions cosmétiques ou dermopharmaceutiques selon l'une quelconque des revendications de 1 à 2 **caractérisées en ce que** l'extrait de *Solanum lycocarpum* est obtenu grâce à une extraction de la matière sèche au moyen des techniques de macération, de lixiviation, de décoction, d'extraction à contre courant, d'extraction à l'aide d'ultrasons, de micro-ondes associée ou non aux solvants.

4.  Compositions cosmétiques ou dermopharmaceutiques selon la revendication 3 **caractérisées en ce que** le solvant d'extraction est choisi parmi le groupe constitué par : l'eau, le propylène glycol, le butylène glycol, la glycérine, les polyéthylènes glycols, les éthers méthyliques ou éthyliques des diglycols, les polyols cycliques, les diglycols éthoxylés ou propoxylés ou tout mélange de ces solvants.

5.  Compositions cosmétiques ou dermopharmaceutiques selon l'une quelconque des revendications de 1 à 4 **caractérisées en ce que** l'extrait de *Solanum lycocarpum* est utilisé soit sous forme liquide soit sous forme sèche obtenue par atomisation, évaporation ou lyophilisation.

6.  Compositions cosmétiques ou dermopharmaceutiques selon l'une quelconque des revendications de 1 à 5 **caractérisées en ce que** la concentration en extrait de *Solanum lycocarpum* est comprise entre 0.1 et 30% (p/p), préférentiellement entre 0.5 et 10% en poids de la composition totale.

7.  Compositions cosmétiques ou dermopharmaceutiques selon l'une quelconque des revendications de 1 à 6 **caractérisées en ce que** les extraits de *Solanum lycocarpum* sont utilisés dans toute forme galénique employée en cosmétique ou dermopharmacie à savoir les émulsions H/E et E/H, laits, lotions, gels, pommades, huiles corporelles, lotions capillaires, shampooings, savons, sticks et crayons, sprays.

8.  Compositions cosmétiques ou dermopharmaceutiques selon l'une quelconque des revendications de 1 à 7 **caractérisées en ce que** les extraits de *Solanum lycocarpum* sont incorporés dans des vecteurs cosmétiques comme les liposomes, les chylomicrons, les macro-, micro- et nanoparticules ainsi que les macro-, micro- et nanocapsules, ou absorbés sur des polymères organiques poudreux, les talcs, bentonites et autres supports minéraux.

9. Compositions cosmétiques ou dermopharmaceutiques selon l'une quelconque des revendications de 1 à 8 **caractérisées en ce que** les extraits de *Solanum lycocarpum* sont combinés dans les compositions cosmétiques avec tout autre ingrédient habituellement utilisé en cosmétique : lipides d'extraction et ou de synthèse, polymères gélifiants et viscosants, tensioactifs et émulsifiants, principes actifs hydro- ou liposolubles, extraits de plantes, extraits tissulaires, extraits marins.

10. Compositions cosmétiques ou dermopharmaceutiques selon l'une quelconque des revendications de 1 à 9 **caractérisées en ce que** les extraits de *Solanum lycocarpum* sont utilisés dans les applications cosmétiques pour tous les soins de la peau y compris le traitement anti-vieillissement, antirides, anti-inflammatoire, antiradicalaire, dépigmentant, pour l'hydratation et l'effet de lissage, pour le traitement du cuir chevelu et de la peau acnéique.

**Claims**

1. Cosmetic or dermopharmaceutical compositions having anti-ageing activity, promoting or regulating skin cell renewal, protecting against hair loss and having anti-inflammatory and anti-radical activities, **characterised in that** they contain an extract of *Solanum lycocarpum*.

2. Cosmetic or dermopharmaceutical compositions according to claim 1, **characterised in that** the above-mentioned *Solanum lycocarpum* extract is obtained from the whole plant or preferably from the fresh or dry fruits of *Solanum lycocarpum*.

3. Cosmetic or dermopharmaceutical compositions according to either claim 1 or claim 2, **characterised in that** the *Solanum lycocarpum* extract is obtained by extraction from the dry material by techniques of maceration, leaching, decoction, countercurrent extraction, extraction by means of ultrasound, microwaves, which extraction is associated or is not associated with solvents.

4. Cosmetic or dermopharmaceutical compositions according to claim 3, **characterised in that** the extraction solvent is selected from the group constituted by: water, propylene glycol, butylene glycol, glycerol, polyethylene glycols, methyl or ethyl diglycol ethers, cyclic polyols, ethoxylated or propoxylated diglycols, or any mixture of those solvents.

5. Cosmetic or dermopharmaceutical compositions according to any one of claims 1 to 4, **characterised in that** the *Solanum lycocarpum* extract is used either in liquid form or in dry form obtained by atomisation, evaporation or lyophilisation.

6. Cosmetic or dermopharmaceutical compositions according to any one of claims 1 to 5, **characterised in that** the concentration of *Solanum lycocarpum* extract is between 0.1 and 30% (by weight), preferably between 0.5 and 10% by weight of the total composition.

7. Cosmetic or dermopharmaceutical compositions according to any one of claims 1 to 6, **characterised in that** the *Solanum lycocarpum* extracts are used in any galenical form employed in cosmetics or dermopharmacy, that is to say, oil-in-water and water-in-oil emulsions, milks, lotions, gels, ointments, body oils, hair lotions, shampoos, soaps, sticks and pencils, sprays.

8. Cosmetic or dermopharmaceutical compositions according to any one of claims 1 to 7, **characterised in that** the *Solanum lycocarpum* extracts are incorporated in cosmetic carriers, such as liposomes, chylomicrons, macro-, micro- and nanoparticles and also macro-, micro- and nanocapsules, or are absorbed on powdery organic polymers, talcs, bentonites and other mineral supports.

9. Cosmetic or dermopharmaceutical compositions according to any one of claims 1 to 8, **characterised in that** the *Solarium lycocarpum* extracts are combined in the cosmetic compositions with any other ingredient customarily used in cosmetics: lipids obtained by extraction and/or by synthesis, gelling and viscosity-increasing polymers, surfactants and emulsifiers, hydro- or liposoluble active ingredients, plant extracts, tissue extracts, marine extracts.

10. Cosmetic or dermopharmaceutical compositions according to any one of claims 1 to 9, **characterised in that** the *Solanum lycocarpum* extracts are used in cosmetic applications for all types of skin care, including anti-ageing, anti-wrinkle, anti-inflammatory, anti-radical and depigmenting treatment, and for hydration and the smoothing effect, for the treatment of the scalp and skin affected by acne.

**Patentansprüche**

1. Kosmetische oder dermatopharmazeutische Zusammensetzungen mit Wirksamkeit gegen Alterung, die zelluläre Erneuerung der Haut fördernd oder regulierend, vor Haarausfall schützend und antiphlogistische und gegen Radikale gerichtete Wirkungen besitzend, **dadurch gekennzeichnet, dass** sie einen Extrakt von *Solanum lycocarpum* enthalten.

**2.** Kosmetische oder dermatopharmazeutische Zusammensetzungen nach Anspruch 1, **dadurch gekennzeichnet, dass** der genannte Extrakt von *Solanum lycocarpum* aus der ganzen Pflanze oder vorzugsweise aus den frischen oder trockenen Früchten von *Solanum lycocarpum* erhalten wird.

**3.** Kosmetische oder dermatopharmazeutische Zusammensetzungen nach einem der Ansprüche 1 bis 2, **dadurch gekennzeichnet, dass** der Extrakt von *Solanum lycocarpum* erhalten wird dank einer Extraktion der Trockensubstanz mittels Techniken der Mazeration, der Auslaugung, der Abkochung, der Extraktion im Gegenstrom, der Extraktion mittels Ultraschall, mittels Mikrowellen, mit Lösungsmitteln verbunden oder nicht.

**4.** Kosmetische oder dermatopharmazeutische Zusammensetzungen nach Anspruch 3, **dadurch gekennzeichnet, dass** das Extraktionslösungsmittel gewählt wird unter der Gruppe bestehend aus: Wasser, Propylenglykol, Butylenglykol, Glycerin, den Polyethylenglykolen, den Methyl- oder Ethylethern der Diglykole, den cyclischen Polyolen, den ethoxylierten oder propoxylierten Diglykolen oder jeder Mischung dieser Lösungsmittel.

**5.** Kosmetische oder dermatopharmazeutische Zusammensetzungen nach einem der Ansprüche 1 bis 4, **dadurch gekennzeichnet, dass** der Extrakt von *Solanum lycocarpum* entweder in flüssiger Form oder in trockener Form, durch Zerstäubung, Verdampfung oder Gefriertrocknung erhalten, verwendet wird.

**6.** Kosmetische oder dermatopharmazeutische Zusammensetzungen nach einem der Ansprüche 1 bis 5, **dadurch gekennzeichnet, dass** die Konzentration an Extrakt von *Solanum lycocarpum* zwischen 0,1 und 30 Gew.-%, vorzugsweise zwischen 0,5 und 10 Gew.-%, der Gesamtzusammensetzung liegt.

**7.** Kosmetische oder dermatopharmazeutische Zusammensetzungen nach einem der Ansprüche 1 bis 6, **dadurch gekennzeichnet, dass** die Extrakte von *Solanum lycocarpum* in jeder in der Kosmetik oder Dermatopharmazie gebrauchten galenischen Form verwendet werden, nämlich den Öl-Wasserund Wasser-Öl-Emulsionen, Milch, Lotionen, Gelen, Pomaden, Körperölen, Haarlotionen, Shampoos, Seifen, Sticks und Stiften, Sprays.

**8.** Kosmetische oder dermatopharmazeutische Zusammensetzungen nach einem der Ansprüche 1 bis 7, **dadurch gekennzeichnet, dass** die Extrakte von *Solanum lycocarpum* in kosmetischen Vektoren wie den Liposomen, den Chylomikronen, den Makro-, Mikro- und Nanopartikeln sowie den Makro-, Mikro- und Nanokapseln eingebaut sind oder auf pulverförmigen organischen Polymeren, Talken, Bentoniten und anderen mineralischen Trägern absorbiert sind.

**9.** Kosmetische oder dermatopharmazeutische Zusammensetzungen nach einem der Ansprüche 1 bis 8, **dadurch gekennzeichnet, dass** die Extrakte von *Solanum lycocarpum* in den kosmetischen Zusammensetzungen mit jedem anderen üblicherweise in der Kosmetik gebrauchten Bestandteil kombiniert sind: extrahierten und/oder synthetischen Lipiden, Gel bildenden und viskos machenden Polymeren, grenzflächenaktiven und emulgierenden Stoffen, wasser- oder fettlöslichen aktiven Prinzipien, Pflanzenextrakten, Gewebeextrakten, Meeresextrakten.

**10.** Kosmetische oder dermatopharmazeutische Zusammensetzungen nach einem der Ansprüche 1 bis 9, **dadurch gekennzeichnet, dass** die Extrakte von *Solanum lycocarpum* in den kosmetischen Anwendungen für alle Behandlungen der Haut, eingeschlossen die Anti-Alterungs-, Anti-Falten-, antiphlogistische, gegen Radikale wirkende, depigmentierende Behandlung, für die Hydratation und die Glättungswirkung, für die Behandlung der behaarten Kopfhaut und der Akne-Haut gebraucht werden.